**Europäisches Patentamt**

**European Patent Office**

**Office européen des brevets**

(11) Veröffentlichungsnummer: **0 133 880**
**B1**

(12) # EUROPÄISCHE PATENTSCHRIFT

(45) Veröffentlichungstag der Patentschrift:
07.12.88

(51) Int. Cl.⁴: **A 61 K 31/70**

(21) Anmeldenummer: **84106388.6**

(22) Anmeldetag: **05.06.84**

(54) Verwendung von Sucralfat zur Bekämpfung von Emesis und/oder Diarrhoe.

(30) Priorität: **20.06.83 DE 3322078**

(43) Veröffentlichungstag der Anmeldung:
**13.03.85 Patentblatt 85/11**

(45) Bekanntmachung des Hinweises auf die Patenterteilung:
**07.12.88 Patentblatt 88/49**

(84) Benannte Vertragsstaaten:
**AT BE CH DE FR GB IT LI NL SE**

(56) Entgegenhaltungen:
CHEMICAL ABSTRACTS, Band 90, Nr. 3, 15. Januar 1979, Seite 3, Nr. 15891y, Columbus, Ohio, USA; H. MATSUO: "Ulcerlmin"
CHEMICAL ABSTRACTS, TENTH COLLECTIVE INDEX, Bänder 86-95, 1977-1981, American Chemical Society, Columbus, Ohio, USA
CHEMICAL ABSTRACTS, Band 99, Nr. 23, 5. Dezember 1983, Seite 100, Nr. 187725z, Columbus, Ohio, USA; I.N. MARKS et al.: "Sucralfate: New aspects in therapy of ulcers and lesions. Second international sucralfate symposium together with the world congress of gastroenterology in Stockholm"
Gastroenterology 84, no. 5, part 2, page 1119, May 1983
"Drug Responses in Man", a Ciba Foundation Volume, edited by G. WOLSTENHOLME and R. PORTER, London, 1967, Titelseite und Seiten 6, 10 und 11

(73) Patentinhaber: **Merck Patent Gesellschaft mit beschränkter Haftung, Frankfurter Strasse 250, D-6100 Darmstadt (DE)**

(72) Erfinder: **Steiner, Kurt, Dr., Breitensteinstrasse 90, D-8018 Grafing (DE)**

(56) Entgegenhaltungen: (Fortsetzung)
L. LAUNOY, "Leçons sur la Toxicité", Paris, 1935, Titelseite und Seiten 73 bis 75
M. FRIMMER, "Pharmakologie und Toxikologie", Stuttgart, 1969, Titelseite und Seiten 27 und 28
T. KOPPANYI and M.A. AVERY, Clin. Pharmacol. Ther., Band 7, 1966, Seiten 250, 251, 255 und 256
LÜTHGEN, Der parktische Tierarzt, Band 4, 1987, Seite 66
R.T. Williams, "Detoxication Mechanisms", London, 1959, Titelseite und Seiten 732-735.

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Bekämpfung von Emesis und/oder Diarrhoe in der Veterinärmedizin.

Emesis und Diarrhoe kommen in der Veterinärmedizin häufig vor, insbesondere auch in der Kleintierpraxis. Es fehlt jedoch an geeigneten, leicht anwendbaren Mitteln für diese Indikationen.

Der Erfindung lag die Aufgabe zugrunde, neue Wege zur Bekämpfung von Emesis und/oder Diarrhoe in der Veterinärmedizin aufzufinden und geeignete leicht anwendbare Mittel dafür zur Verfügung zu stellen. Diese Aufgabe wurde durch die vorliegende Erfindung gelöst.

Es wurde gefunden, dass das gut verträgliche Sucralfat sich für die angegebenen Zwecke vorzüglich eignet.

Gegenstand der Erfindung ist dementsprechend die Verwendung von Sucralfat zur Herstellung eines verterinärmedizinischen Arzneimittels mit der Indikation Emesis und/oder Diarrhoe.

Sucralfat (Ulcogant®) ist ein basisches Aluminium-Saccharose-Sulfat. Es ist aus der DE-OS 15 68 346 bekannt und wird in der Humanmedizin zur Beschleunigung der Ulkusabheilung und Linderung der Beschwerden bei Ulcus ventriculi et duodeni verwendet. Eine Wirkung des Sucralfats gegen Emesis und/oder Diarrhoe ist bisher nicht bekannt.

Erfindungsgemäss können in erster Linie Kleintiere behandelt werden, vorzugsweise Hunde und Katzen, ferner z.B. Kälber, Schweine (Ferkel) und Zootiere. Das Sucralfat wird zweckmässig in Einzeldosen zwischen 0,5 und 5, vorzugsweise zwischen 1 und 2 g verabfolgt; das entspricht etwa 25 bis 250 mg/kg pro Dosis. Katzen und kleine Hunde erhalten zweckmässig jeweils etwa 1 g, grössere Hunde jeweils etwa 2 g als Einzeldosis. Diese Einzeldosen werden vorzugsweise ein- bis dreimal täglich, insbesondere zweimal täglich – morgens und abends – über einen Zeitraum von 1 bis 3 Tagen, allenfalls auch länger bis zum Verschwinden der Symptome, verabreicht, zweckmässig in Form einer wässerigen Suspension von Sucralfat-Granulat, die mit Hilfe einer Spritze buccal – hinter die Lefzen – gegeben wird.

Bei dieser Art der Anwendung kann von den handelsüblichen Formen des Sucralfats ausgegangen werden. Alle anderen in der Veterinärmedizin üblichen Anwendungsformen sind jedoch ebenfalls geeignet, z.B. Pulver, Kapseln, Tabletten, Dragees oder Säfte.

Beispiel 1

Ein Pudel litt seit neun Tagen an teilweise sehr heftigem Erbrechen zusammen mit Durchfall. Es wurden zweimal je 1 g Ulcogant®-Granulat in Form einer wässerigen Suspension hinter die Lefzen im Abstand von 8 Stunden verabreicht. Innerhalb von 24 Stunden waren die Symptome verschwunden.

Beispiel 2

Einem Hund, der eine durch Schneefressen induzierte Gastroenteritis entwickelte, wurden drei Tage lang jeweils morgens und abends je zwei g Ulcogant®-Granulat als wässerige Suspension oral verabreicht. Er war danach beschwerdefrei.

Beispiel 3

Eine Katze, die an Diarrhoe litt, wurde zwei Tage lang dreimal täglich mit jeweils 0,5 g Ulcogant®-Granulat in Form einer wässerigen Suspension oral behandelt. Danach war die gastrointestinale Störung behoben.

**Patentanspruch**

Verwendung von Sucralfat zur Herstellung eines veterinärmedizinischen Arzneimittels mit der Indikation Emesis und/oder Diarrhoe.

**Claim**

Use of sucralfate for controlling emesis and/or diarrhoea in veterinary medicine.

**Revendication**

Utilisation du Sucralfat pour la préparation d'un médicament vétérinaire pour le traitement des vomissements et/ou des diarrhées.